(19) **Europäisches Patentamt / European Patent Office / Office européen des brevets**

(11) **EP 1 551 391 B1**

(12) **EUROPEAN PATENT SPECIFICATION**

(45) Date of publication and mention
of the grant of the patent:
**27.06.2007 Bulletin 2007/26**

(51) Int Cl.:
*A61K 31/375* (2006.01)     *A61K 31/194* (2006.01)
*A61K 31/4704* (2006.01)

(21) Application number: **03769898.2**

(22) Date of filing: **10.10.2003**

(86) International application number:
**PCT/JP2003/013042**

(87) International publication number:
**WO 2004/035044 (29.04.2004 Gazette 2004/18)**

(54) **DRY SYRUP PREPARATION OF PROCATEROL COMPRISING ASCORBIC ACID AND CITRIC ACID**

TROCKENSIRUP-ZUBEREITUNG VON PROCATEROL MIT ASCORBINSÄURE UND ZITRONENSÄURE

PREPARATION DE PROCATEROL SOUS FORME DE SIROP SEC CONTENANT DE L'ACIDE ASCORBIQUE ET DE L'ACIDE CITRIQUE

(84) Designated Contracting States:
**IT PT**

(30) Priority: **15.10.2002 JP 2002299894**

(43) Date of publication of application:
**13.07.2005 Bulletin 2005/28**

(73) Proprietor: **OTSUKA PHARMACEUTICAL CO., LTD.**
**Chiyoda-ku**
**Tokyo 101-8535 (JP)**

(72) Inventors:
• **OKAMURA, Akio**
**Itano-gun, Tokushima 771-1231 (JP)**

• **SEKI, Hajime**
**Tokushima-shi, Tokushima 770-0045 (JP)**

(74) Representative: **von Kreisler, Alek et al**
**Deichmannhaus am Dom,**
**Postfach 10 22 41**
**50462 Köln (DE)**

(56) References cited:
**CH-A- 388 536**

• **URBANSKY ET AL: "Ascorbic acid reduction of residual active chlorine in potable water prior to halocarboxylate determination" J. ENVIRON. MONIT., vol. 2, 2000, pages 253-256, XP002268233**

**EP 1 551 391 B1**

**Description**

Technical Field

The present invention relates to a dry syrup preparation of procaterol.

Background Art

Procaterol (chemical name: ($\pm$)-erythro-8-hydroxy-5-[1-hydroxy-2-(isopropylamino)butyl]carbostyril) has a bronchodilating activity (cf. JP-A-60-120864) and has widely been used for the treatment of respiratory diseases such as bronchitis, bronchial asthma, etc.

It has been used in various formulations such as tablets, powders, granules, capsules, but it is desirable to formulate in syrups or dry syrups for administration to children. The syrup or dry syrup preparation is suitable not only for children but also aged persons in view of easier administration. Particularly, dry syrup preparation is advantageous because it is easily weighed and packaged and further it is convenient for carrying.

CH 388 536 A discloses the use of ascorbinic acid for preventing the oxidation of compounds by chlorine solved in tap water.

Urbansky et al. disclose in J. Environ. Monit., vol. 2, 2000, pages 253-256 that citric acid can be used to induce a synergistic effect on the prevention of oxidation when combined with asorbinic acid.

From the above-mentioned viewpoints, it is desired to develop a dry syrup preparation of procaterol. The present inventors have intensively studied to find a new dry syrup preparation of procaterol. During the studies, the present inventors have noted that a dry syrup preparation of procaterol is prepared in a conventional formulation using conventional carriers and excipients, it has the following problem.

In order to take a dry syrup, it is usually required to be diluted with water. Although the dilution of the dry syrup is usually done with a tap water, the tap water contains usually free residual chlorine, and owing to the free residual chlorine, the active ingredient procaterol or a salt thereof is immediately decomposed when diluted with the tap water. Thus, when a dry syrup of procaterol or a salt thereof is prepared into a syrup by diluting with a tap water before administration, the active ingredient procaterol or a salt thereof is mostly decomposed, and hence, the preparation is not practically used.

Thus, it is desirable to find a new type of dry syrup preparation of procaterol which can be diluted with a tap water without the problem of decomposition of the active procaterol or a salt thereof by the tap water.

Disclosure of Invention

Under the circumstance as mentioned above, the present inventors have further intensively studied to find a new dry syrup of procaterol or a salt thereof having no problem of decomposition of the active procaterol or a salt thereof even by diluting with a tap water. As a result, it has been found that the problem can be solved by incorporating ascorbic acids to the dry syrup preparation because the ascorbic acids can inhibit the undesirable decomposition of procaterol or a salt thereof with a tap water. The present inventors have further found that the dry syrup of procaterol or a salt thereof prepared by incorporating ascorbic acids has still a problem in storage stability, because the ascorbic acids are decomposed during storage of the preparation for a long period of time, which retains still a problem of decomposition of the active procaterol or a salt thereof by a tap water.

Then, the present inventors have further studied and have found that when a dry syrup preparation comprising procaterol or a salt thereof and ascorbic acids is further incorporated with a specific amount of citric acid, the storage stability of ascorbic acids is significantly improved, and hence, even when such a dry syrup preparation is stored for a long period of time, the dry syrup preparation can be stably maintained and hence can be diluted with a tap water to give a stable syrup preparation which is largely prevented from the undesirable decomposition of the active procaterol or a salt thereof, and then, the present invention has been accomplished.

The present invention includes the following embodiments.

1. A dry syrup preparation comprising at least one active ingredient selected from procaterol or a salt thereof, an ascorbic acid, and citric acid, wherein citric acid is contained in an amount of 0.001 to 0.03 % by weight based on the whole weight of the preparation.

2. The dry syrup preparation of procaterol or a salt thereof according to the above item 1, wherein citric acid is contained in an amount of 0.001 to 0.01 % by weight based on the whole weight of the preparation.

3. The dry syrup preparation of procaterol or a salt thereof according to the above item 2, wherein citric acid is contained in an amount of 0.002 to 0.005 % by weight based on the whole weight of the preparation.

4. The dry syrup preparation of procaterol according to any one of the above items 1 to 3, wherein the ascorbic acid is contained in an amount of 0.03 to 10 % by weight based on the whole weight of the preparation.

5. A method for preventing decomposition of procaterol by a tap water in a dry syrup preparation of procaterol comprising as an active ingredient procaterol or a salt thereof, which comprises incorporating an ascorbic acid and citric acid into the preparation,

wherein the amount of citric acid is 0.001 to 0.03 % by weight, based on the whole weight of the preparation.

6. The method according to the above item 5, wherein the ascorbic acid is incorporated in an amount of 0.03 to 10 % by weight and citric acid is incorporated in an amount of 0.001 to 0.03 % by weight, based on the whole weight of the preparation.

Best Mode for Carrying Out the Invention

The dry syrup preparation of procaterol of the present invention contains as an active ingredient at least one compound selected from procaterol or a salt thereof.

The salt of procaterol includes conventional pharmaceutically acceptable acid addition salts, for example, inorganic acid salts such as hydrochloride, sulfate, nitrate, or hydrobromide, or organic acid salts such as oxalate, maleate, fumarate, citrate, tartrate, or lactate. Preferable salt of procaterol are hydrochloride, i.e. procaterol hydrochloride. These procaterol or a salt thereof may also be in the form of a hydrate, for example, procaterol hydrochloride hemihydrate.

The procaterol or a salt thereof may be contained in any amount in the dry syrup preparation, but is usually contained in an amount of 0.001 to 0.1 % by weight, preferably 0.002 to 0.05 % by weight, more preferably 0.005 to 0.01 % by weight, based on the whole weight of the preparation.

The ascorbic acids to be incorporated into the preparation include, for example, ascorbic acid or isoascorbic acid (i.e. erythorbic acid). Among them, ascorbic acid is preferable.

The ascorbic acids may be contained in any amount in the dry syrup preparation, but is usually contained in an amount of 0.03 to 10 % by weight, preferably 0.05 to 5 % by weight, more preferably 0.1 to 2 % by weight, based on the whole weight of the preparation.

The dry syrup preparation of the present invention shall be incorporated with citric acid. Procaterol or a salt thereof to be contained in the preparation of the present invention is unstable and is easily hydrolyzed with water, but when ascorbic acids are incorporated, the decomposition of procaterol or a salt thereof can be prevented or inhibited. On the other hand, the ascorbic acids are also inferior in storage stability and hence, the preparation incorporated with ascorbic acids is kept for a long period of time, the ascorbic acids are decomposed to change to other compounds. As a result, the stabilization effects of procaterol or a salt of said ascorbic acids are decreased. In order to improve such a defect, citric acid is further incorporated into the preparation, by which the stability of ascorbic acids is significantly improved to give the desired dry syrup preparation of procaterol having excellent storage stability. It is to be noted, however, that when the citric acid is incorporated in too much amount, it results unfavorably in a tendency of decomposition of procaterol to deform into threo isomer thereof. Thus, the citric acid may usually be contained in the preparation in an amount of 0.001 to 0.03 % by weight, preferably 0.001 to 0.01 % by weight, more preferably 0.002 to 0.005 % by weight, based on the whole weight of the preparation.

The preparation of the present invention may further contain, in addition to the above components, excipients such as saccharides, sugar alcohols. The amount of the excipients is the remaining amount obtained by deducting the total amount of the active compound, ascorbic acids and citric acid from the whole amount of the preparation.

The saccharides include any known saccharides, for example, glucose, fructose, sucrose, and the like. The sugar alcohols include any known sugar alcohols, for example, mannitol, sorbitol, xylitol, erythritol, and the like. Preferred saccharide is sucrose, and preferred sugar alcohol is mannitol.

These excipients may be used alone or in a mixture of two or more kinds thereof.

The preparation of the present invention may optionally contain other additives such as a pH adjuster, a fluidizing agent. The pH adjuster includes any known agents, for example, succinic acid, oxalic acid, malic acid, and the like. The fluidizing agent includes any known agents, for example, light silicic anhydride, and the like. These additives may be added in place of a part of the excipients as mentioned above.

The dry syrup preparation of the present invention may be prepared by mixing the components as mentioned above in any order. The preparation of the present invention may also be prepared by dissolving or dispersing the above components in water (e.g. a purified water) or other solvent, and drying by known drying method, such as spontaneous drying or drying with hot air.

The dry syrup preparation of the present invention may usually be diluted with water (e.g. a tap water) by 1 to 100 folds dilution, preferably 2 to 20 folds dilution, when used.

The dry syrup preparation of procaterol of the present invention can be kept stably without decomposition of the active procaterol or a salt thereof even by diluting with a tap water, and the procaterol or a salt thereof can be retained in 90 % by weight or more, preferably 95 % by weight or more.

Examples

The dry syrup preparation of procaterol of the present invention is illustrated by the following examples but should not be construed to be limited thereto.

Examples 1 and 2 and Reference Example 1

The components of the following Table 1 are mixed in the amounts as shown therein to give dry syrup preparations of Examples 1 and 2 and Reference Example 1.

Table 1

| Ingredients | | Ref. Ex. 1 | Example 1 | Example 2 |
|---|---|---|---|---|
| Procaterol hydrochloride | (mg) | 0.05 | 0.05 | 0.05 |
| Purified sucrose | (mg) | 993.95 | 993.93 | 993.75 |
| Ascorbic acid | (mg) | 5.00 | 5.00 | 5.00 |
| Citric acid | (mg) | 0.00 | 0.02 | 0.20 |
| Light silicic anhydride | (mg) | 1.00 | 1.00 | 1.00 |
| Total amount | (mg) | 1000.00 | 1000.00 | 1000.00 |

Experiment 1

Each dry syrup preparation obtained in the above Examples 1 and 2 and Reference Example 1 were kept at 50°C for 3 months, and thereafter, the content of ascorbic acid in the preparation was measured. On the basis of the contents (100 %) of ascorbic acid immediately after preparing, the remaining rate (%) of ascorbic acid in the preparation was calculated by the following equation:

$$\text{Remaining rate (\%) of ascorbic acid} = \frac{\text{Content of ascorbic acid in preparation after kept for 3 months}}{\text{Content of ascorbic acid in preparation immediately after preparing}} \times 100$$

The results are shown in Table 2.

Table 2

| | Ref. Ex. 1 | Example 1 | Example 2 |
|---|---|---|---|
| Remaining rate of ascorbic acid (%) | 63.1 | 87.2 | 97.1 |

As is clear from Table 2, by incorporating citric acid (in Examples 1 and 2), the remaining rate (%) of ascorbic acid can be increased in comparison with the preparation without citric acid (Reference Example 1). It is also clear that with increasing the amount of citric acid, the remaining rate (%) of ascorbic acid can be highly increased.

Experiment 2

Each dry syrup preparation obtained in the above Examples 1 and 2 and Reference Example 1 were kept at 50°C for 3 months, and thereafter, the content of procaterol hydrochloride in the preparation was measured. On the basis of the contents (100 %) of procaterol hydrochloride immediately after preparing, the remaining rate (%) of procaterol hydrochloride in the preparation was calculated by the following equation:

$$\text{Remaining rate (\%) of procaterol HCl} = \frac{\text{Content of procaterol HCl in preparation after kept for 3 months}}{\text{Content of procaterol HCl in preparation immediately after preparing}} \times 100$$

The results are shown in Table 3.

Besides, it was also measured what rate (%) of the procaterol hydrochloride was decomposed and changed into threo isomer and aldehyde compound in the preparation after kept for 3 months. The results are also shown in Table 3.

Table 3

|  | Ref. Ex. 1 | Example 1 | Example 2 |
|---|---|---|---|
| Remaining rate of procaterol HCl (%) | 97.61 | 98.78 | 94.47 |
| Threo isomer (%) | 0.63 | 0.82 | 5.21 |
| Aldehyde compound (%) | 1.46 | 0.40 | 0.32 |

As is clear from Table 3, by adding citric acid, the decomposition of procaterol hydrochloride into aldehyde compound during storage at a high temperature can be inhibited, but when citric acid is added in such a higher amount than 0.02 % by weight like in Example 2, the decomposition rate of procaterol hydrochloride into the threo isomer is increased. On the other hand, as in Example 1, the amount of citric acid in the preparation is 0.02 % by weight or less, the dry syrup preparation shows higher remaining rate of procaterol hydrochloride and less decomposition into threo isomer and aldehyde compound.

Reference Preparation 1

Procaterol hydrochloride (0.05 mg) was mixed with purified sucrose (500.00 mg) and D-mannitol (499.95 mg) to give a dry syrup preparation.

Reference Preparation 2

Procaterol hydrochloride (0.05 mg) was mixed with ascorbic acid (0.10 mg), purified sucrose (500.00 mg) and D-mannitol (499.85 mg) to give a dry syrup preparation.

Reference Preparation 3

Procaterol hydrochloride (0.05 mg) was mixed with ascorbic acid (0.30 mg), purified sucrose (500.00 mg) and D-mannitol (499.65 mg) to give a dry syrup preparation.

Reference Preparations 4 to 6

In the same manner as described in Reference Preparation 3 excepting changing the amount of ascorbic acid to 1.00 mg, 3,00 mg and 5,00 mg and also changing the amount of D-mannitol to those obtained by deducting the corresponding increased amount of ascorbic acid, respectively, there were obtained various dry syrup preparations.

Those preparations of Reference Preparations 1 to 6 are summarized in Table 4.

Table 4

|  |  | Reference Preparations | | | | | |
|---|---|---|---|---|---|---|---|
|  |  | 1 | 2 | 3 | 4 | 5 | 6 |
| Procaterol Hydrochloride | (mg) | 0.05 | 0.05 | 0.05 | 0.05 | 0.05 | 0.05 |
| Purified sucrose | (mg) | 500.00 | 500.00 | 500.00 | 500.00 | 500.00 | 500.00 |
| D-Mannitol | (mg) | 499.95 | 499.85 | 499.65 | 498.95 | 496.95 | 494.95 |

(continued)

| | | Reference Preparations | | | | | |
|---|---|---|---|---|---|---|---|
| | | 1 | 2 | 3 | 4 | 5 | 6 |
| Ascorbic acid | (mg) | 0.00 | 0.10 | 0.30 | 1.00 | 3.00 | 5.00 |
| Total amount | (mg) | 1000.00 | 1000.00 | 1000.00 | 1000.00 | 1000.00 | 1000.00 |

Experiment 3

Each dry syrup preparation obtained in the above Reference Preparations 1 to 6 was diluted with a tap water (10 ml) to prepare a syrup preparation. After the dilution, the content of procaterol hydrochloride in the syrup preparation was measured and the remaining rate (%) of procaterol hydrochloride in the syrup preparation was calculated. The results are shown in Table 5.

Table 5

| | Reference Preparations | | | | | |
|---|---|---|---|---|---|---|
| | 1 | 2 | 3 | 4 | 5 | 6 |
| Remaining rate of Procaterol HCI (%) | 0.0 | 10.2 | 97.6 | 98.8 | 99.2 | 100.0 |

As is clear from Table 5, in the preparation of Reference Preparation 1 which did not contain ascorbic acid, when it was dissolved in a tap water, procaterol hydrochloride was wholly decomposed. It is also clear from Table 5 that with increasing the amount of ascorbic acid, the remaining rate (%) of procaterol hydrochloride in the syrup preparation could be made higher. That is, the larger the amount of the ascorbic acid, the more the decomposition of procaterol hydrochloride by a tap water was inhibited.

Thus, the preparation of the present invention of Example 1 can maintain high remaining rate of ascorbic acid even after kept at 50°C for 3 months with less composition of procaterol hydrochloride into threo isomer, and hence the preparation of the present invention is particularly preferable preparation.

Industrial Applicability

The dry syrup preparation of procaterol of the present invention is stable and the active procaterol or a salt thereof can be kept without being decomposed even by diluting with a tap water. That is, by incorporating citric acid into a dry syrup preparation comprising procaterol or a salt thereof and ascorbic acids, the stability of ascorbic acids is improved, and thereby the ascorbic acids can be kept stably even when stored for a long period of time, and then, the procaterol or a salt thereof contained in the preparation can be prevented from decomposition even by dilution with a tap water. Thus, the dry syrup preparation of procaterol of the present invention is very useful as a medicament, particularly as a medicament for children and aged persons.

**Claims**

1.    A dry syrup preparation of procaterol, which comprises procaterol or a salt thereof, an ascorbic acid and citric acid, wherein citric acid is contained in an amount of 0.001 to 0.03 % by weight based on the whole weight of the preparation.

2.    The dry syrup preparation of procaterol according to claim 1, wherein the amount of citric acid is in the range of 0.001 to 0.01 % by weight based on the whole weight of the preparation.

3.    The dry syrup preparation of procaterol according to claim 2, wherein the amount of citric acid is in the range of 0.002 to 0.005 % by weight based on the whole weight of the preparation.

4.    The dry syrup preparation of procaterol according to any one of claims 1 to 3, wherein the ascorbic acid is contained in an amount of 0.03 to 10 % by weight based on the whole weight of the preparation.

5.    A method for preventing decomposition of procaterol by a tap water in a dry syrup preparation of procaterol comprising as an active ingredient procaterol or a salt thereof, which comprises incorporating an ascorbic acid and citric acid into the preparation,
wherein the amount of citric acid is 0.001 to 0.03 % by weight, based on the whole weight of the preparation.

**6.** The method according to the above item 5, wherein the ascorbic acid is incorporated in an amount of 0.03 to 10 % by weight and citric acid is incorporated in an amount of 0.001 to 0.03 % by weight, based on the whole weight of the preparation.

**Patentansprüche**

**1.** Trockensirupppräparat von Procaterol, das Procaterol oder ein Salz davon, Ascorbinsäure und Zitronensäure umfasst, wobei Zitronensäure in einer Menge von 0,001 bis 0,03 Gew.-% enthalten ist, bezogen auf das Gesamtgewicht des Präparats.

**2.** Trockensirupppräparat von Procaterol gemäß Anspruch 1, wobei die Menge an Zitronensäure im Bereich von 0,001 bis 0,01 Gew.-% liegt, bezogen auf das Gesamtgewicht des Präparats.

**3.** Trockensiruppräparat von Procaterol gemäß Anspruch 2, wobei die Menge an Zitronensäure im Bereich von 0,002 bis 0,005 Gew.-% liegt, bezogen auf das Gesamtgewicht des Präparats.

**4.** Trockensiruppräparat von Procaterol gemäß einem der Ansprüche 1 bis 3, wobei die Ascorbinsäure in einer Menge von 0,03 bis 10 Gew.-% enthalten ist, bezogen auf das Gesamtgewicht des Präparats.

**5.** Verfahren zur Prävention der Zersetzung von Procaterol durch Leitungswasser in einem Trockensirupppräparat von Procaterol, das Procaterol oder ein Salz davon als Wirkstoff umfasst, wobei das Verfahren das Einarbeiten von Ascorbinsäure und Zitronensäure in das Präparat umfasst, wobei die Menge der Zitronensäure 0,001 bis 0,03 Gew.-% beträgt, bezogen auf das Gesamtgewicht des Präparats.

**6.** Verfahren gemäß dem obigen Anspruch 5, wobei die Ascorbinsäure in einer Menge von 0,03 bis 10 Gew.-% eingearbeitet ist und die Zitronensäure in einer Menge von 0,001 bis 0,03 Gew.-% eingearbeitet ist, bezogen auf das Gesamtgewicht des Präparats.

**Revendications**

**1.** Préparation de sirop sec de procatérol, qui comprend du procatérol ou un de ses sels, un acide ascorbique et de l'acide citrique, l'acide citrique étant contenu en une quantité de 0,001 à 0,03 % en masse par rapport à la masse totale de la préparation.

**2.** Préparation de sirop sec de procatérol selon la revendication 1, dans laquelle la quantité d'acide citrique se situe dans le domaine de 0,001 à 0,01 % en masse par rapport à la masse totale de la préparation.

**3.** Préparation de sirop sec de procatérol selon la revendication 2, dans laquelle la quantité d'acide citrique se situe dans le domaine de 0,002 à 0,005 % en masse par rapport à la masse totale de la préparation.

**4.** Préparation de sirop sec de procatérol selon l'une quelconque des revendications 1 à 3, dans laquelle l'acide ascorbique est contenu en une quantité de 0,03 à 10 % en masse par rapport à la masse totale de la préparation.

**5.** Procédé de prévention de la décomposition du procatérol par l'eau du robinet dans une préparation de sirop sec de procatérol comprenant comme ingrédient actif du procatérol ou un de ses sels, qui comprend l'incorporation d'un acide ascorbique et d'acide citrique dans la préparation, la quantité d'acide citrique étant de 0,001 à 0,03 % en masse par rapport à la masse totale de la préparation.

**6.** Procédé selon le point 5 ci-dessus, dans lequel l'acide ascorbique est incorporé en une quantité de 0,03 à 10 % en masse et l'acide citrique est incorporé en une quantité de 0,001 à 0,03 % en masse par rapport à la masse totale de la préparation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60120864 A

- CH 388536 A

**Non-patent literature cited in the description**

- **URBANSKY et al.** *J. Environ. Monit.,* 2000, vol. 2, 253-256